# EUROPEAN PATENT APPLICATION

(11) **EP 4 810 609 A1**
(43) Date of publication of application: **23.09.2026**
(21) Application number: 25725740.2
(22) Date of filing: 17.01.2025
(51) Int. Cl.: C12N 15/12, A61K 38/18, A61K 48/00, A61P 9/10, A61P 11/00, A61P 17/02, A61P 25/02, A61P 27/02, C07H 1/00, C07H 21/02, C12N 15/10

(54) **NUCLEIC ACID, DRUG COMPOSITION, AND METHOD OF PRODUCING NUCLEIC ACID**

(30) Priority: 17.11.2023 JP 2023196002
(71) Applicant: AnGes, Inc., Ibaraki-shi, Osaka 567-0085 (JP)
(72) Inventor: SHIBUI, Tatsuro, Ibaraki-shi, Osaka 567-0085 (JP)
(74) Representative: Cabinet Becker et Associés
(86) International application number: PCT/JP2025/001478
(87) International publication number: WO 2025/105506

(57) **Abstract**

A nucleic acid includes a modified protein-coding region, in which a total proportion of uracil residues and adenine residues in the modified protein-coding region is reduced relative to a corresponding protein-coding region before modification. In addition, a method for producing the nucleic acid is provided.

## Description

### TECHNICAL FIELD

The present invention relates to a nucleic acid, a drug composition, and a method for producing a nucleic acid.

Priority is claimed on Japanese Patent Application No. 2023-196002, filed November 17, 2023, the content of which is incorporated herein by reference.

### BACKGROUND ART

In recent years, the practical application of nucleic acid drugs such as nucleic acids for gene therapy and mRNA vaccines has been promoted. As nucleic acids for gene therapy, the use of messenger RNA (mRNA) in addition to DNA drugs such as DNA plasmids and viral vectors has been proposed. However, the use of exogenously supplied RNA has been limited due to the problem of immunogenicity.

In order to reduce the immunogenicity of RNA, it has been proposed to reduce a contained amount of uridine by replacing uridine in mRNA with modified uridine or the like (Patent Document 1).

### Citation List

### Patent Document

Patent Document 1: Published Japanese Translation No. 2018-525410 of the PCT International Publication

### SUMMARY OF INVENTION

### Technical Problem

In nucleic acid drugs containing a protein-coding region, there is a demand for suppressing cytotoxic activity caused by immunogenicity or the like, as well as for improving a production amount of the protein.

Therefore, an object of the present invention is to provide a nucleic acid having low cytotoxic activity and an improved production amount of a protein, a drug composition containing the nucleic acid, and a method for producing the nucleic acid. Solution to Problem

The present invention includes the following aspects.
[1] A nucleic acid including a modified protein-coding region, in which a total proportion of uracil residues and adenine residues in the modified protein-coding region is reduced relative to a corresponding protein-coding region before modification.
[2] The nucleic acid according to [1], in which at least a portion of codons included in the protein-coding region before modification are replaced with codons encoding the same amino acid and having a lower proportion of uracil and adenine.
[3] The nucleic acid according to [1] or [2], in which at least a portion of codons included in the protein-coding region before modification and having uracil as a first base are replaced with codons encoding the same amino acid and having adenine as a first base.
[4] The nucleic acid according to any one of [1] to [3], in which at least a portion of codons included in the protein-coding region before modification are replaced with codons encoding the same amino acid and more frequently used in a cell into which the nucleic acid is to be introduced.
[5] The nucleic acid according to any one of [1] to [4], in which at least a portion of uracil residues contained in the nucleic acid are residues derived from a modified uracil nucleotide.
[6] The nucleic acid according to [5], in which the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.
[7] The nucleic acid according to any one of [1] to [6], in which the nucleic acid is an mRNA.
[8] The nucleic acid according to any one of [1] to [7], in which the protein-coding region encodes a hepatocyte growth factor.
[9] The nucleic acid according to [8], in which a nucleotide sequence of the protein-coding region is a nucleotide sequence set forth in SEQ ID NO: 1.
[10] A drug composition containing the nucleic acid according to any one of [1] to [9].
[11] A drug composition for treating or preventing an ischemic disease, containing the nucleic acid according to [8] or [9].
[12] The drug composition according to [11], in which the ischemic disease is chronic arterial occlusion, chronic obstructive pulmonary disease, interstitial pneumonia, acute lung injury, ophthalmic disease, optic nerve injury, or intractable skin ulcer.
[13] A method for producing a nucleic acid including a protein-coding region, the method including: (a) a step of determining a nucleotide sequence of the protein-coding region according to the following principles (a1) to (a4), (a1) codon-optimizing the protein-coding region for a cell into which the nucleic acid is to be introduced, (a2) selecting a codon to reduce a total proportion of uracil residues and adenine residues in the protein-coding region, (a3) in codons encoding the same amino acid, when alternatives for a first base of a codon are uracil and adenine, selecting a codon in which the first base is adenine, and (a4) in codons encoding the same amino acid, when alternatives for a third base of a codon include guanine and cytosine, selecting a codon in which the third base is guanine or cytosine and which is more frequently used in the cell; and (b) a step of producing a nucleic acid including the protein-coding region of the nucleotide sequence determined in the step (a).
[14] The method according to [13], in which, in the step (b), a modified uracil nucleotide is used as a uracil nucleotide.
[15] The method according to [14], in which the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.

### Advantageous Effects of Invention

According to the present invention, there are provided a nucleic acid having low cytotoxic activity and an improved production amount of a protein, a drug composition containing the nucleic acid, and a method for producing the nucleic acid.

### BRIEF DESCRIPTION OF DRAWINGS

[FIG. 1] Showing a Cap1 structure among 5' cap structures of mRNA.
[FIG. 2] Showing a structure of mRNA for human HGF production (AG-mRNA) synthesized in Examples.
[FIG. 3] Showing a production amount of human HGF at each nucleic acid introduction amount.
[FIG. 4] Showing a dose-response regression line of a production amount of human HGF in AG-mRNA, created from results of AG-mRNA shown in FIG. 3.
[FIG. 5] Showing cell viability at each nucleic acid introduction amount.
[FIG. 6] Showing an overview of a test schedule of a continuous exposure test of a nucleic acid.
[FIG. 7] Showing a temporal change in human HGF concentration in a culture supernatant in the continuous exposure test of the nucleic acid.
[FIG. 8] Showing a temporal change in intracellular human HGF concentration in the continuous exposure test of the nucleic acid.
[FIG. 9] Showing an overview of a test schedule of a 48-hour exposure test of the nucleic acid.
[FIG. 10] Showing a daily amount of human HGF released from inside the cells to the outside of the cells in the 48-hour exposure test of the nucleic acid.
[FIG. 11] Showing a cumulative amount of human HGF released from inside the cells to the outside of the cells from 48 hours to 120 hours of culture in the 48-hour exposure test of the nucleic acid.

### DESCRIPTION OF EMBODIMENTS

The terms "polynucleotide" and "nucleic acid" are used interchangeably to refer to a polymer of nucleotides, in which the nucleotides are linked by phosphodiester bonds. The "polynucleotide" and the "nucleic acid" may be deoxyribonucleic acid (DNA), ribonucleic acid (RNA), or may be composed of a combination of DNA and RNA. The "polynucleotide" and the "nucleic acid" may refer to a polymer of natural nucleotides, a polymer of natural nucleotides and non-natural nucleotides (a non-natural nucleotide being an analog of a natural nucleotide and/or a nucleotide in which at least one moiety selected from a base moiety, a sugar moiety, and a phosphate moiety is modified), or a polymer of non-natural nucleotides.

A nucleotide sequence of the "polynucleotide" or the "nucleic acid" is described in a generally accepted one-letter code unless otherwise explicitly stated. Unless otherwise explicitly stated, the nucleotide sequence is described from a 5' side toward a 3' side.

Nucleotide residues constituting the "polynucleotide" or the "nucleic acid" may be described simply as adenine, thymine, cytosine, guanine, or uracil, or by their one-letter codes (A, T, C, G, U).

The terms "polypeptide", "peptide", and "protein" are used interchangeably to refer to a polymer of amino acids, in which the amino acids are linked by an amide bond. The "polypeptide", the "peptide", or the "protein" may be a polymer of natural amino acids, may be a polymer of natural amino acids and non-natural amino acids (chemical analogs, modified derivatives, and the like of natural amino acids), or may be a polymer of non-natural amino acids.

An amino acid sequence of the "polypeptide", the "peptide", or the "protein" is described in a generally accepted one-letter code or three-letter code unless otherwise explicitly stated. Unless otherwise explicitly stated, the amino acid sequence is described from an N-terminal side toward a C-terminal side.

The "gene" refers to a polynucleotide including at least one open reading frame (ORF) encoding a specific protein. The gene may include both an exon and an intron.

The "protein-coding region" refers to a region of a nucleic acid that is translated into a protein. More specifically, the "protein-coding region" refers to a region in mRNA that is translated into a protein and is located between a start codon and a stop codon, and a corresponding region in the DNA that serves as a template for the mRNA. The protein-coding region may be divided into a plurality of exons.

The "residue" refers to a structure (structural unit) derived from each monomer in a polymer formed by bonding monomers.

The "uracil residue" refers to a residue derived from a uracil nucleotide or a modified uracil nucleotide. The "adenine residue" refers to a residue derived from an adenine nucleotide or a modified adenine nucleotide. The "cytosine residue" refers to a residue derived from a cytosine nucleotide or a modified cytosine nucleotide. The "guanine residue" refers to a residue derived from a guanine nucleotide or a modified guanine nucleotide. The "thymine residue" refers to a residue derived from a thymine nucleotide or a modified thymine nucleotide.

The "uracil nucleotide" refers to a nucleotide including uracil as a base. The "adenine nucleotide" refers to a nucleotide including adenine as a base. The "cytosine nucleotide" refers to a nucleotide including cytosine as a base. The "guanine nucleotide" refers to a nucleotide including guanine as a base. The "thymine nucleotide" refers to a nucleotide including thymine as a base.

The "modified uracil nucleotide" refers to a nucleotide including uracil or modified uracil as a base, in which at least one moiety selected from the group consisting of a base (uracil), a sugar (ribose), and a phosphate (triphosphate) moiety is modified. The "modified adenine nucleotide" refers to a nucleotide including adenine or modified adenine as a base, in which at least one moiety selected from the group consisting of a base (adenine), a sugar (ribose or deoxyribose), and a phosphate (triphosphate) moiety is modified. The "modified cytosine nucleotide" refers to a nucleotide including cytosine or modified cytosine as a base, in which at least one moiety selected from the group consisting of a base (cytosine), a sugar (ribose or deoxyribose), and a phosphate (triphosphate) moiety is modified. The "modified guanine nucleotide" refers to a nucleotide including guanine or modified guanine as a base, in which at least one moiety selected from the group consisting of a base (guanine), a sugar (ribose or deoxyribose), and a phosphate (triphosphate) moiety is modified. The "modified thymine nucleotide" refers to a nucleotide including thymine or modified thymine as a base, in which at least one moiety selected from the group consisting of a base (thymine), a sugar (deoxyribose), and a phosphate (triphosphate) moiety is modified.

In a nucleic acid, a residue derived from the modified uracil nucleotide has the same function as a residue derived from uridine triphosphate. In a nucleic acid, a residue derived from the modified adenine nucleotide has the same function as a residue derived from adenosine triphosphate or deoxyadenosine triphosphate. In a nucleic acid, a residue derived from the modified cytosine nucleotide has the same function as a residue derived from cytidine triphosphate or deoxycytidine triphosphate. In a nucleic acid, a residue derived from the modified guanine nucleotide has the same function as a residue derived from guanosine triphosphate or deoxyguanosine triphosphate. A residue derived from the modified thymine nucleotide has the same function as a residue derived from deoxythymidine triphosphate.

As the modified uracil nucleotide, the modified adenine nucleotide, the modified cytosine nucleotide, the modified guanine nucleotide, and the modified thymine nucleotide, known nucleotides can be used without particular limitation.

The "wild-type" refers to that which occurs naturally. For example, a wild-type nucleic acid refers to a naturally occurring nucleic acid. Examples of the wild-type nucleic acid include a nucleic acid isolated from a naturally occurring cell of an organism or a virus. For example, a wild-type gene may be a gene isolated from the wild-type nucleic acid. Examples of the wild-type protein include a protein isolated from a naturally occurring cell of an organism or a virus.

The "codon optimization" refers to replacing at least one codon of an original nucleotide sequence with a codon more frequently used in a target species while maintaining the original amino acid sequence. A codon usage frequency table is readily available, for example, from the "Codon Usage Database" (www.kazusa.or.jp/codon/) provided by the Kazusa DNA Research Institute. For example, a codon can be optimized using the codon usage frequency table. A computer algorithm for codon-optimizing a specific sequence for expression in a specific animal species is also known. The computer algorithm for codon-optimizing is available, for example, from Gene Forge (Aptagen, LLC; Jacobus, PA).

### (Nucleic acid)

A first aspect of the present invention is a nucleic acid (hereinafter, also referred to as "modified nucleic acid") including a protein-coding region that has been modified (hereinafter, also referred to as "modified protein-coding region"). In one embodiment, the modified nucleic acid has a reduced total proportion of uracil residues and adenine residues in the modified protein-coding region relative to a corresponding protein-coding region before modification (hereinafter, also referred to as "protein-coding region before modification").

The modified nucleic acid may be RNA or DNA. In one embodiment, the modified nucleic acid may be an mRNA, or may be a DNA including at least one ORF. When the modified nucleic acid is DNA, the modified nucleic acid may be in a form of a vector such as a plasmid vector and a viral vector. The modified nucleic acid is preferably an mRNA. In the following description, when the modified nucleic acid is DNA, uracil (U) is replaced with thymine (T). Alternatively, an mRNA transcribed from the DNA may be an mRNA that includes the modified protein-coding region described below.

### <Modified protein-coding region>

The modified nucleic acid includes at least one modified protein-coding region. The modified protein-coding region is one in which a nucleotide sequence of the protein-coding region before modification is modified. The protein-coding region before modification may be a protein-coding region of a wild-type nucleic acid, or may be a protein-coding region of a wild-type nucleic acid to which a change has been made.

A protein encoded by the protein-coding region before modification is not particularly limited, and may be a desired protein. The above-described protein may be a wild-type protein, or may be a modified protein obtained by modifying a wild-type protein. The above-described protein may be a protein derived from any organism (including animals, plants, protists, bacteria, and viruses), and may be any type of protein.

Examples of the protein encoded by the protein-coding region before modification include a hepatocyte growth factor (HGF). An amino acid sequence of human HGF is shown in SEQ ID NO: 2.

The modified protein-coding region encodes the same protein as the protein-coding region before modification. The modified protein-coding region preferably encodes the same amino acid sequence as the protein-coding region before modification.

The modified nucleic acid may include two or more modified protein-coding regions, and may include two or more types of modified protein-coding regions. When the modified nucleic acid is DNA, the modified protein-coding region may be divided into two or more exons by one or more introns.

The modified protein-coding region satisfies the following condition (i). In addition, the modified protein-coding region preferably satisfies one or more of the following conditions (ii) and (iii).
(i) The total proportion of uracil residues and adenine residues is reduced relative to the protein-coding region before modification.
(ii) Among codons included in the protein-coding region before modification, at least a portion of codons having uracil as a first base are replaced with codons encoding the same amino acid and having adenine as a first base.
(iii) At least a portion of the codons included in the protein-coding region before modification are replaced with codons encoding the same amino acid and more frequently used in a cell into which the modified nucleic acid is to be introduced.

### Condition (i):

The modified protein-coding region is modified such that the total proportion of uracil residues and adenine residues is reduced relative to the protein-coding region before modification. In the modified protein-coding sequence, for example, the total proportion of uracil residues and adenine residues is reduced by, for example, 5% or more, 10% or more, 15% or more, or 20% or more relative to the protein-coding region before modification.

Such modification can be achieved, for example, by replacing at least a portion of codons included in a wild-type protein-coding region with codons having a lower proportion of uracil and adenine. In one embodiment, 60% or more, 70% or more, 80% or more, 90% or more, 95% or more, or 100% of the codons containing either or both of uracil and adenine among the codons in the protein-coding region before modification are replaced with codons having a lower proportion of uracil and adenine in the modified protein-coding region.

Table 1 shows a codon table.

**[Table 1]**

| First base | Second base | | | | | | | | Third base |
|---|---|---|---|---|---|---|---|---|---|
| | U | | C | | A | | G | | |
| U | UUU | Phe | UCU | Ser | UAU | Tyr | UGU | Cys | U |
| | UUC | | UCC | | UAC | | UGC | | C |
| | UUA | Leu | UCA | | UAA | STOP | UGA | STOP | A |
| | UUG | | UCG | | UAG | | UGG | Trp | G |
| C | CUU | Leu | CCU | Pro | CAU | His | CGU | Arg | U |
| | CUC | | CCC | | CAC | | CGC | | C |
| | CUA | | CCA | | CAA | Gln | CGA | | A |
| | CUG | | CCG | | CAG | | CGG | | G |
| A | AUU | Ile | ACU | Thr | AAU | Asn | AGU | Ser | U |
| | AUC | | ACC | | AAC | | AGC | | C |
| | AUA | | ACA | | AAA | Lys | AGA | Arg | A |
| | AUG | Met | ACG | | AAG | | AGG | | G |
| G | GUU | Val | GCU | Ala | GAU | Asp | GGU | Gly | U |
| | GUC | | GCC | | GAC | | GGC | | C |
| | GUA | | GCA | | GAA | Glu | GGA | | A |
| | GUG | | GCG | | GAG | | GGG | | G |

Examples of the codons having a low proportion of uracil and adenine include codons shown in Table 2. In one embodiment, at least a portion of the codons included in the protein-coding region before modification are converted to the codons shown in Table 2 in the modified protein-coding region. In one embodiment, the codons shown in Table 2 are used for 60% or more, 70% or more, 80% or more, 90% or more, 95% or more, or 100% of the codons included in the modified protein-coding region. It is preferable that the codons shown in Table 2 are used for all of the codons included in the modified protein-coding region.

**[Table 2]**

| Amino acid | Codon that can be used to satisfy condition (i) |
|---|---|
| Ala | GCC, GCG |
| Gly | GGC, GGG |
| Val | GUC, GUG |
| Asp | GAC |
| Glu | GAG |
| Ile | AUC |
| Thr | ACC, ACG |
| Asn | AAC |
| Lys | AAG |
| Ser | UCC, UCG, AGC |
| Arg | CGC, CGG |
| Leu | CUG, CUC |
| Pro | CCG, CCC |
| His | CAC |
| Gln | CAG |
| Phe | UUC |
| Tyr | UAC |
| Cys | UGC |
| Trp | UGG |
| Met | AUG |

### Condition (ii):

In the modified protein-coding region, among the codons included in the protein-coding region before modification, at least a portion of codons having uracil as a first base may be replaced with codons encoding the same amino acid and having adenine as a first base. In one embodiment, 60% or more, 70% or more, 80% or more, 90% or more, 95% or more, or 100% of codons having uracil as a first base among codons included in a wild-type protein-coding region are replaced with codons encoding the same amino acid and having adenine as a first base in the modified protein-coding region.

Table 3 shows codons that can be used to satisfy the conditions (i) and (ii). In one embodiment, at least a portion of the codons included in the protein-coding region before modification are converted to the codons shown in Table 3 in the modified protein-coding region. In one embodiment, the codons shown in Table 3 are used for 60% or more, 70% or more, 80% or more, 90% or more, 95% or more, or 100% of the codons included in the modified protein-coding region. It is preferable that the codons shown in Table 3 are used for all of the codons included in the modified protein-coding region.

**[Table 3]**

| Amino acid | Codon that can be used to satisfy conditions (i) and (ii) |
|---|---|
| Ala | GCC, GCG |
| Gly | GGC, GGG |
| Val | GUC, GUG |
| Asp | GAC |
| Glu | GAG |
| Ile | AUC |
| Thr | ACC, ACG |
| Asn | AAC |
| Lys | AAG |
| Ser | AGC |
| Arg | CGC, CGG |
| Leu | CUG, CUC |
| Pro | CCG, CCC |
| His | CAC |
| Gln | CAG |
| Phe | UUC |
| Tyr | UAC |
| Cys | UGC |
| Trp | UGG |
| Met | AUG |

### Condition (iii):

In the modified protein-coding region, at least a portion of the codons included in the protein-coding region before modification may be replaced with codons encoding the same amino acid and more frequently used in a cell into which the modified nucleic acid is to be introduced (hereinafter, also referred to as an "introduction target cell").

For example, when the introduction target cell of the modified nucleic acid is a human cell, in the modified protein-coding region, at least a portion of the codons included in the protein-coding region before modification may be replaced with codons more frequently used in humans.

A codon usage frequency table in the introduction target cell is easily available, for example, from "Codon Usage Database" (www.kazusa.or.jp/codon/) provided by Kazusa DNA Research Institute.

For example, in codons encoding the same amino acid, when a codon having guanine as a third base has a higher usage frequency in the introduction target cell than a codon having cytosine as a third base, the codon having guanine as the third base may be selected. In this case, in the modified protein-coding region, among the codons included in the protein-coding region before modification, at least a portion of codons having cytosine as a third base may be replaced with codons encoding the same amino acid and having guanine as a third base. In this case, 60% or more, 70% or more, 80% or more, 90% or more, 95% or more, or 100% of codons having cytosine as a third base among the codons included in the protein-coding region before modification may be replaced with codons encoding the same amino acid and having guanine as a third base in the modified protein-coding region.

For example, in codons encoding the same amino acid, when a codon having cytosine as a third base has a higher usage frequency in the introduction target cell than a codon having guanine as a third base, the codon having cytosine as the third base may be selected. In this case, in the modified protein-coding region, among the codons included in the protein-coding region before modification, at least a portion of codons having guanine as a third base may be replaced with codons encoding the same amino acid and having cytosine as a third base. In this case, 60% or more, 70% or more, 80% or more, 90% or more, 95% or more, or 100% of codons having guanine as a third base among the codons included in the protein-coding region before modification may be replaced with codons encoding the same amino acid and having cytosine as a third base in the modified protein-coding region.

Table 4 shows codons that can be used in humans to satisfy the conditions (i) to (iii). When the introduction target cell is a human cell, at least a portion of the codons included in the protein-coding region before modification may be converted to the codons shown in Table 4 in the modified protein-coding region. When the introduction target cell is a human cell, the codons shown in Table 4 may be used for 60% or more, 70% or more, 80% or more, 90% or more, 95% or more, or 100% of the codons included in the modified protein-coding region. When the introduction target cell is a human cell, it is preferable that the codons shown in Table 4 are used for all of the codons included in the modified protein-coding region.

**[Table 4]**

| Amino acid | Codon that can be used to satisfy conditions (i) to (iii) (in human) |
|---|---|
| Ala | GCC |
| Gly | GGC |
| Val | GUG |
| Asp | GAC |
| Glu | GAG |
| Ile | AUC |
| Thr | ACC |
| Asn | AAC |
| Lys | AAG |
| Ser | AGC |
| Arg | CGC |
| Leu | CUG |
| Pro | CCC |
| His | CAC |
| Gln | CAG |
| Phe | UUC |
| Tyr | UAC |
| Cys | UGC |
| Trp | UGG |
| Met | AUG |

In one embodiment, the modified protein-coding region may be a protein-coding region obtained by codon-optimizing the protein-coding region before modification for the introduction target cell, and then modifying the codon-optimized protein-coding region to satisfy the above-described conditions (i) and (ii). For example, the modified protein-coding region may take the form of any of the following embodiments.
(a) A modified protein-coding region in which a protein-coding region before modification is codon-optimized for an introduction target cell, and at least a portion of codons included in the codon-optimized protein-coding region are replaced with codons encoding the same amino acid and having a lower proportion of uracil and adenine.
(b) A modified protein-coding region in which a protein-coding region before modification is codon-optimized for an introduction target cell, and at least a portion of codons having uracil as a first base among codons included in the codon-optimized protein-coding region are replaced with codons encoding the same amino acid and having adenine as a first base.

The modified protein-coding region according to (a) or (b), in which at least a portion of codons having guanine or cytosine as a third base are further replaced with codons encoding the same amino acid and having guanine or cytosine as a third base, which are more frequently used in the cell.

In the above (a) to (c), the "at least a portion" includes 60% or more of the corresponding total codons, and may be 70% or more, 80% or more, 90% or more, 95% or more, or 100% of the corresponding total codons.

The modified nucleic acid includes at least one open reading frame (ORF), and the modified protein-coding region may be included in the ORF.

As a specific example of a nucleotide sequence of the modified protein-coding region, the nucleotide sequence thereof encoding human HGF is shown in SEQ ID NO: 1. In SEQ ID NO: 1, "T" represents a uracil residue in RNA and a thymine residue in DNA. "A" represents an adenine residue, "C" represents a cytosine residue, and "G" represents a guanine residue. An amino acid sequence of human HGF encoded by the modified protein-coding region including the nucleotide sequence set forth in SEQ ID NO: 1 is shown in SEQ ID NO: 2.

The amino acid sequence of the human HGF is not limited to that set forth in SEQ ID NO: 2, and may be, for example, any of the following amino acid sequences (i) to (iii).
(i) An amino acid sequence including the amino acid sequence set forth in SEQ ID NO: 2.
(ii) An amino acid sequence in which one or a few amino acids in the amino acid sequence set forth in SEQ ID NO: 2 have been mutated.
(iii) An amino acid sequence having 80% or more sequence identity with the amino acid sequence set forth in SEQ ID NO: 2.

In the above (i), the amino acid sequence may include an additional amino acid sequence at either or both of an N-terminus and a C-terminus of the amino acid sequence set forth in SEQ ID NO: 2. A length of the added amino acid sequence is not particularly limited, and examples thereof include 1 to 100 amino acids, 1 to 50 amino acids, 1 to 30 amino acids, 1 to 20 amino acids, 1 to 10 amino acids, or 1 to 5 amino acids.

In the above (ii), examples of "a few" include 2 to 20, 2 to 15, 2 to 10, 2 to 9, 2 to 8, 2 to 7, 2 to 6, 2 to 5, 2 to 4, 2 and 3, or 2. The "mutation" may be any of deletion, substitution, addition, and insertion, or a combination thereof.

In the above (iii), the sequence identity may be 85% or more, 90% or more, 91% or more, 92% or more, 93% or more, 94% or more, 95% or more, 96% or more, 97% or more, 98% or more, or 99% or more.

In the amino acid sequences of (i) to (iii) described above, a protein consisting of the amino acid sequence is preferably a functional HGF. The "functional HGF" refers to a protein that functions as HGF. The functional HGF preferably exhibits an HGF activity (hepatocyte proliferation-promoting activity and the like) equal to or greater than that of the protein consisting of the amino acid sequence set forth in SEQ ID NO: 2.

In one embodiment, the modified protein-coding region may encode the amino acid sequence of any of the above (i) to (iii).

### <Other regions>

The modified nucleic acid may include other regions in addition to the above-described modified protein-coding region. For example, when the modified nucleic acid is an mRNA, examples of the other regions include a 5' Cap, a 5' untranslated region (5' UTR), a 3' untranslated region (3' UTR), and a poly(A) tail. When the modified nucleic acid is DNA, examples of the other regions include a promoter and a terminator.

### Cap structure:

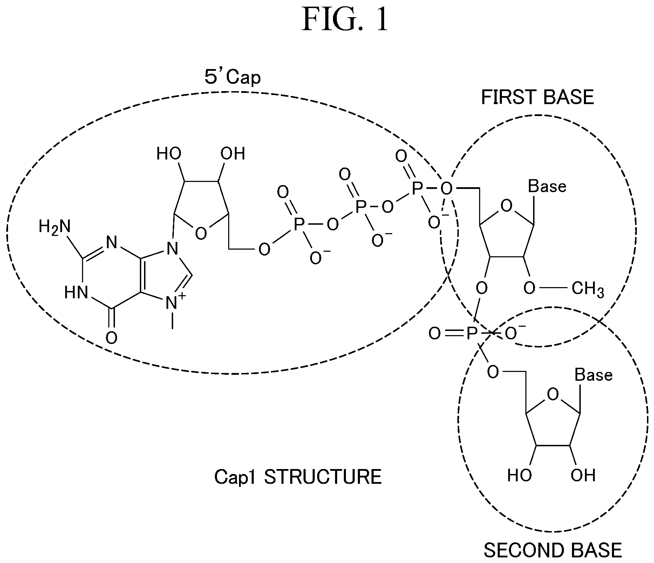

When the modified nucleic acid is an mRNA, it may include a 5' Cap at a 5' end. Examples of the 5' Cap include structures such as a Cap0 structure, a Cap1 structure, and a Cap2 structure. The cap structure is usually a 7-methylguanine ribonucleotide, and is linked through a 5'-triphosphate to the 5' position of the first nucleotide of the mRNA chain in the 5'-to-3' direction, that is, a first cap-proximal nucleotide. In the Cap0 structure, riboses of first and second cap-proximal nucleotides of the mRNA both contain a 2'-hydroxyl. In the Cap1 structure, a ribose of a first cap-proximal nucleotide of the mRNA contains a 2'-methoxy, and a ribose of a second nucleotide contains a 2'-hydroxyl (see FIG. 1). In the Cap2 structure, riboses of first and second cap-proximal nucleotides of the mRNA both contain a 2'-methoxy.

The modified nucleic acid preferably includes the 5' Cap, and more preferably includes the Cap1 structure as the 5' Cap.

The Cap structure can be incorporated into the 5' end of the mRNA during transcription by a known method. For example, the Cap1 structure can be incorporated into the mRNA by co-transcription using CleanCap(TM) AG (TriLink Biotechnologies).

The Cap structure may be added to a chemically synthesized RNA or after transcription of the mRNA using a capping enzyme.

### 5' UTR and 3' UTR:

When the modified nucleic acid is an mRNA, the modified nucleic acid may include either one or both of the 5' UTR and the 3' UTR. The 5' UTR and the 3' UTR may be those of the mRNA from which the protein-coding region before modification is derived, or those of a different mRNA. The nucleotide sequence of the 5' UTR may have 100% sequence identity with a nucleotide sequence of a wild-type 5' UTR, or at least a portion thereof may be modified. The nucleotide sequence of the 3' UTR may have 100% sequence identity with a nucleotide sequence of a wild-type 3' UTR, or at least a portion thereof may be modified.

Examples of the mRNA from which the 5' UTR and the 3' UTR are derived include the mRNA from which the protein-coding region before modification is derived, and, for example, mRNAs of globin (for example, human alpha globin (HBA), human beta globin (HBB), and Xenopus beta globin (XBG)), bovine growth hormone, cytomegalovirus (CMV), mouse Hba-a1, hydroxysteroid 17-beta dehydrogenase 4 (for example, HSD17B4 and HSD), albumin, heat shock protein 90 (Hsp90), glyceraldehyde-3-phosphate dehydrogenase (GAPDH), beta-actin, alpha-tubulin, tumor protein (p53), and epidermal growth factor receptor (EGFR), without being limited thereto.

The modified nucleic acid may include a Kozak sequence. The Kozak sequence may affect translation initiation and the total amount of protein produced from the mRNA. The Kozak sequence includes a methionine codon that can function as a start codon. A minimal Kozak sequence is NNNRUGN (N is any nucleotide residue, and R is a purine residue (A or G)). In the above sequence, the first N is preferably A or G, and the second N is preferably G. In one embodiment, the Kozak sequence is RNNRUGN, NNNRUGG, RNNRUGG, RNNAUGN, NNNAUGG, or RNNAUGG.

### Poly(A) tail:

When the modified nucleic acid is an mRNA, the modified nucleic acid may include a poly(A) tail at a 3' end. The poly(A) tail may include at least 8 consecutive adenine nucleotide sequences, but may also include one or more non-adenine nucleotide residues (for example, G, C, U). Examples of a length of the poly(A) tail include 10 to 500 nucleotides, 30 to 300 nucleotides, and 60 to 250 nucleotides.

Examples of the structure of the mRNA include the following. **In** the following, the 5' UTR may include only the Kozak sequence.
(1) An mRNA including the 5' Cap, the 5' UTR, the modified protein-coding region (or the ORF including the modified protein-coding region), the 3' UTR, and the poly(A) tail in this order from the 5' side.
(2) An mRNA including the 5' Cap, the 5' UTR, the modified protein-coding region (or the ORF including the modified protein-coding region), and the poly(A) tail in this order from the 5' side.
(3) An mRNA including the 5' UTR, the modified protein-coding region (or the ORF including the modified protein-coding region), the 3' UTR, and the poly(A) tail in this order from the 5' side.
(4) An mRNA including the 5' UTR, a disulfide bond-cleaving enzyme-coding region, and the poly(A) tail in this order from the 5' side.
(5) An mRNA including the 5' Cap, the 5' UTR, the modified protein-coding region (or the ORF including the modified protein-coding region), and the 3' UTR in this order from the 5' side.
(6) An mRNA including the 5' Cap, the 5' UTR, and the modified protein-coding region (or the ORF including the modified protein-coding region) in this order from the 5' side.
(7) An mRNA including the 5' UTR, the modified protein-coding region (or the ORF including the modified protein-coding region), and the 3' UTR in this order from the 5' side.
(8) An mRNA including the 5' UTR and the modified protein-coding region (or the ORF including the modified protein-coding region) in this order from the 5' side.
(9) An mRNA including the 5' Cap, the modified protein-coding region (or the ORF including the modified protein-coding region), the 3' UTR, and the poly(A) tail in this order from the 5' side.
(10) An mRNA including the 5' Cap, the modified protein-coding region (or the ORF including the modified protein-coding region), and the poly(A) tail in this order from the 5' side.

### <Modified nucleotide>

The modified nucleic acid may include a modified nucleotide. The "modified nucleotide" refers to a nucleotide in which at least one moiety selected from the group consisting of a base, a sugar, and a phosphate group is modified. The modified nucleotide is not particularly limited, and a known one can be used.

In the modified nucleic acid, it is preferable that at least a portion of uracil residues are residues derived from a modified uracil nucleotide. By using the modified uracil nucleotide, immunogenicity of the modified nucleic acid can be suppressed. In one embodiment, 50% or more, 60% or more, 70% or more, 80% or more, 90% or more, 95% or more, or 100% of the uracil residues contained in the modified nucleic acid are residues derived from the modified uracil nucleotide. It is preferable that all of the uracil residues contained in the modified nucleic acid are residues derived from the modified uracil nucleotide.

The modified uracil nucleotide is not particularly limited, and a known one can be used. Specific examples of the modified uracil nucleotide include, but are not limited to, 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate. In one embodiment, the modified uracil nucleotide is one in which a base (uracil) is modified (modified uracil). In one embodiment, the modified uracil nucleotide can be selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 5-bromouridine-5'-triphosphate, 5-iodouridine-5'-triphosphate, and 4-thiouridine-5'-triphosphate. In one embodiment, the modified uracil nucleotide is 5-methoxyuridine-5'-triphosphate.

One type of the modified uracil nucleotide may be used alone, or two or more types thereof may be used in combination.

By having the characteristics as described above, the modified nucleic acid has reduced cytotoxic activity as compared with the nucleic acid before modification, and improves the amount of protein produced when introduced into a cell. Therefore, it can be suitably used as an agent for causing a target cell to produce a desired protein.

### (Drug composition)

A second aspect of the present invention is a drug composition. In one embodiment, the drug composition contains the above-described modified nucleic acid according to the first aspect.

In one embodiment, the drug composition may contain a pharmaceutically acceptable carrier. The "pharmaceutically acceptable carrier" means a carrier that does not inhibit the physiological activity of the active ingredient and does not exhibit substantial toxicity to the administration subject. The phrase "does not exhibit substantial toxicity" means that the component does not exhibit toxicity to the administration subject at a dosage usually used. In the drug composition according to the present embodiment, the pharmaceutically acceptable carrier is a carrier that does not inhibit the function of the modified nucleic acid according to the first aspect, and does not exhibit substantial toxicity to the administration subject. The pharmaceutically acceptable carrier encompasses any known pharmaceutically acceptable component that is typically considered as an inactive ingredient. Examples of the pharmaceutically acceptable carrier include, but are not limited to, solvents, diluents, vehicles, excipients, glidants, binders, granulating agents, dispersing agents, suspending agents, wetting agents, lubricants, disintegrants, solubilizers, stabilizers, emulsifiers, and fillers. One type of the pharmaceutically acceptable carrier may be used alone, or two or more types thereof may be used in combination. Specific examples of the pharmaceutically acceptable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropyl cellulose, gelatin, gum arabic, polyethylene glycol, sucrose, and starch; disintegrants such as starch, carboxymethyl cellulose, hydroxypropyl starch, sodium starch glycolate, sodium hydrogen carbonate, calcium phosphate, and calcium citrate; lubricants such as magnesium stearate, Aerosil, talc, and sodium lauryl sulfate; flavoring agents such as citric acid, menthol, glycyrrhizin ammonium salt, glycine, and orange powder; preservatives such as sodium benzoate, sodium hydrogen sulfite, methylparaben, and propylparaben; stabilizers such as citric acid, sodium citrate, and acetic acid; suspending agents such as methylcellulose, polyvinylpyrrolidone, and aluminum stearate; dispersing agents such as surfactants; diluents such as water and physiological saline; and base waxes such as cacao butter, polyethylene glycol, and white kerosene.

The drug composition may contain other components in addition to the above-described components. The other components are not particularly limited, and those commonly used in the pharmaceutical field can be used without particular limitation. Examples of the other components include pharmaceutical additives other than those described above. Examples of the pharmaceutical additive include, but are not limited to, preservatives (for example, antioxidants), chelating agents, flavoring agents, sweeteners, thickeners, buffers, and coloring agents. The drug composition may contain an active ingredient other than the modified nucleic acid. Examples of the active ingredient include, but are not limited to, antiviral agents, antibiotics, anti-inflammatory agents, antipyretics, and analgesics. One type of the other components may be used alone, or two or more types thereof may be used in combination.

The drug composition may contain a nucleic acid introduction reagent to promote the introduction of the modified nucleic acid into a cell. Examples of the nucleic acid introduction reagent include, but are not limited to, cationic lipids, cationic liposomes, and cationic polymers. Specific examples of the nucleic acid introduction reagent include, but are not limited to, Lipofectin (trade name, Invitrogen), Lipofectamine (trade name, Invitrogen), Transfectam (trade name, Promega), DOTAP (trade name, Roche Applied Science), dioctadecylamidoglycyl spermine (DOGS), L-dioleoyl phosphatidyl-ethanolamine (DOPE), dimethyldioctadecyl-ammonium bromide (DDAB), N,N-di-n-hexadecyl-N,N-dihydroxyethylammonium bromide (DHDEAB), N-n-hexadecyl-N,N-dihydroxyethylammonium bromide (HDEAB), Polybrene, and poly(ethyleneimine) (PEI).

A dosage form of the drug composition is not particularly limited, and may be a dosage form generally used as a drug preparation. The drug composition according to the present embodiment may be an oral preparation or a parenteral preparation, but is preferably a parenteral preparation. Examples of the oral preparation include tablets, coated tablets, pills, powders, granules, capsules, syrups, fine granules, liquids, drops, and emulsions. Examples of the parenteral preparation include injections, suppositories, nasal drops, enteral agents, and inhalants. The drug compositions of these dosage forms can be formulated according to a conventional method (for example, a method described in the Japanese Pharmacopoeia). The drug composition is preferably a parenteral preparation, and more preferably an injection.

The administration route of the drug composition according to the present embodiment is not particularly limited, and the drug composition can be administered by an oral or parenteral route; but parenteral administration is preferable. Examples of the administration route for parenteral administration include intravenous administration, intranasal administration, subcutaneous administration, intradermal administration, intramuscular administration, intraperitoneal administration, and enteral administration. The administration route is preferably subcutaneous administration, intradermal administration, or intramuscular administration.

The drug composition can be administered in a therapeutically effective amount of the modified nucleic acid. The "therapeutically effective amount" means an amount of a drug effective for treatment or prevention of a target disease. For example, the therapeutically effective amount of the modified nucleic acid may be an amount effective to produce an effective amount of the protein encoded by the modified nucleic acid in a cell. The therapeutically effective amount may be appropriately determined depending on symptoms, body weight, age, and sex of a patient, a dosage form of the drug composition, an administration method, and the like. For example, the drug composition can be administered in a single dose of 0.01 to 1000 mg per 1 kg of body weight of the administration subject, as the modified nucleic acid. The above-described dose may be 0.05 to 500 µg, 0.1 to 200 µg, 0.5 to 100 µg, or 1 to 50 µg.

The drug composition may be administered once or repeatedly. In the case of repeated administration, the administration interval may be appropriately determined depending on symptoms, body weight, age, and sex of the patient, the type of the modified nucleic acid, the dosage form of the drug composition, the administration method, and the like. The administration interval can be, for example, every few hours, 2 to 3 times a day, once every 1 to 5 days, once a week, once a month, once every few months, or the like.

An applicable disease can be selected for the drug composition according to the type of protein encoded by the modified protein-coding region of the modified nucleic acid. In one embodiment, the modified protein-coding region of the modified nucleic acid encodes a protein effective for treating or preventing a target disease. When the modified protein-coding region encodes HGF, the drug composition can be used as a drug composition for treating or preventing an ischemic disease. Examples of the ischemic disease include, for example, chronic arterial occlusion, chronic obstructive pulmonary disease, interstitial pneumonia, acute lung injury, ophthalmic disease, optic nerve injury, and intractable skin ulcers.

The subject to be administered with the drug composition is not particularly limited. The subject to be administered with the drug composition is preferably a mammal, and may be a human or a non-human mammal. Examples of the non-human mammal include, but are not limited to, primates (monkeys, chimpanzees, gorillas, and the like), rodents (mice, hamsters, rats, and the like), rabbits, dogs, cats, cows, goats, sheep, and horses. The subject to be administered is preferably a biological species from which the protein encoded by the modified protein-coding region of the modified nucleic acid is derived. For example, in a case where the modified protein-coding region encodes a human protein, the subject to be administered with the drug composition is preferably a human.

### (Method for producing nucleic acid)

A third aspect of the present invention is a method for producing a nucleic acid including a protein-coding region. The method according to the present embodiment includes the following steps (a) and (b):
(a) a step of determining a nucleotide sequence of the protein-coding region according to the following principles,
(a1) codon-optimizing the protein-coding region for a cell into which the nucleic acid is to be introduced,
(a2) selecting a codon to reduce a total proportion of uracil residues and adenine residues in the protein-coding region, and
(a3) in codons encoding the same amino acid, when alternatives for a first base of a codon are uracil and adenine, selecting a codon in which the first base is adenine; and
(b) a step of producing a nucleic acid including the protein-coding region of the nucleotide sequence determined in the step (a).

### <Step (a)>

In the step (a), the nucleotide sequence of the protein-coding region is determined according to the above-described principles (a1) to (a3). As the protein encoded by the protein-coding region, a desired protein can be selected. Based on the amino acid sequence of the protein, the nucleotide sequence of the protein-coding region is determined according to the above-described principles (a1) to (a3).

In the principle (a1), the codons can be selected as described in the condition (iii) in "<Modified protein-coding region>" of the section of "(Nucleic acid)" above.

In the principle (a2), the codons can be selected as described in the condition (i) in "<Modified protein-coding region>" of the section of "(Nucleic acid)" above. For example, the codons shown in Table 2 can be selected.

In the principle (a3), the codons can be selected as described in the condition (ii) in "<Modified protein-coding region>" of the section of "(Nucleic acid)" above. For example, the codons shown in Table 3 can be selected.

When the introduction target cell is a human, the codons shown in Table 4 can be selected as the codons according to the principles (a1) to (a3).

### <Step (b)>

In the step (b), a nucleic acid including the protein-coding region consisting of the nucleotide sequence determined in the step (a) is produced.

A method for producing the nucleic acid is not particularly limited, and a known method can be used. The nucleic acid may include another region in addition to the protein-coding region. When the nucleic acid is an mRNA, it may include at least one or more selected from the group consisting of a 5' Cap, a 5' UTR, a 3' UTR, and a poly(A) tail. Specific examples thereof and specific examples of the structure of the mRNA are the same as those described in the section of "(Nucleic acid)" above.

When the nucleic acid is RNA, RNA can be produced by a transcription reaction using an RNA polymerase, with a DNA containing the nucleotide sequence of the RNA as a sense strand used as a template. The DNA serving as a template can be obtained by, for example, chemically synthesizing a DNA of approximately 100 to 300 nucleotides and ligating them for DNA assembly.

When producing RNA, a modified nucleotide may be used as a nucleotide used in the transcription reaction. In one embodiment, it is preferable to use a modified uracil nucleotide instead of a uracil nucleotide in the transcription reaction. Examples of the modified uracil nucleotide include those mentioned above. In one embodiment, one in which a base (uracil) is modified (modified uracil) can be used as the modified uracil nucleotide. In one embodiment, 5-methoxyuridine-5'-triphosphate can be used as the modified uracil nucleotide.

One type of the modified uracil nucleotide may be used alone, or two or more types thereof may be used in combination.

When the nucleic acid is an mRNA, a 5' Cap may be added by co-transcriptional capping, or a 5' Cap may be added by post-transcriptional capping. When adding a Cap1 structure as a 5' Cap to a co-transcription method, CleanCap(R) AG (TriLink Biotechnologies) or the like can be used.

After the nucleic acid is synthesized in the step (b), the nucleic acid may be purified. When the nucleic acid is RNA, purification may be performed by DNase treatment, oligo dT purification, or the like.

The nucleic acid according to the first aspect can be produced by the method according to the present embodiment.

The present disclosure includes the following aspects.
[1] A nucleic acid including a modified protein-coding region, in which a total proportion of uracil residues and adenine residues in the modified protein-coding region is reduced relative to a corresponding protein-coding region before modification.
[2] The nucleic acid according to [1], in which at least a portion of codons included in the protein-coding region before modification are replaced with codons encoding the same amino acid and having a lower proportion of uracil and adenine.
[3] The nucleic acid according to [1] or [2], in which at least a portion of codons included in the protein-coding region before modification and having uracil as a first base are replaced with codons encoding the same amino acid and having adenine as a first base.
[4] The modified nucleic acid according to any one of [1] to [3], in which at least a portion of codons in which a third base is guanine among codons included in the protein-coding region before modification are replaced with codons encoding the same amino acid and having cytosine as a third base.
[5] The nucleic acid according to any one of [1] to [4], in which at least a portion of codons in which a third base is cytosine among codons included in the protein-coding region before modification are replaced with codons encoding the same amino acid and having guanine as a third base.
[6] The nucleic acid according to any one of [1] to [5], in which at least a portion of codons included in the protein-coding region before modification are replaced with codons encoding the same amino acid and more frequently used in a cell into which the nucleic acid is to be introduced.
[7] The nucleic acid according to [1], in which the modified protein-coding region is codon-optimized for a cell into which the nucleic acid is to be introduced, and at least a portion of codons included in the codon-optimized protein-coding region are replaced with codons encoding the same amino acid and having a lower proportion of uracil and adenine.
[8] The nucleic acid according to [7], in which at least a portion of codons having uracil as a first base among codons included in the codon-optimized protein-coding region are replaced with codons encoding the same amino acid and having adenine as a first base.
[9] The nucleic acid according to [7] or [8], in which at least a portion of codons having guanine or cytosine as a third base among codons included in the codon-optimized protein-coding region are replaced with codons encoding the same amino acid and having guanine or cytosine as a third base, which are more frequently used in the cell.
[10] The nucleic acid according to any one of [1] to [9], in which at least a portion of the uracil residues contained in the nucleic acid are residues derived from the modified uracil nucleotide.
[11] The nucleic acid according to [10], in which all of the uracil residues included in the nucleic acid are residues derived from the modified uracil nucleotide.
[12] The nucleic acid according to [11], in which the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.
[13] The nucleic acid according to any one of [1] to [12], in which the nucleic acid is an mRNA.
[14] The nucleic acid according to [13], further including a 5' Cap, a 5' untranslated region, and a 3' untranslated region.
[15] The nucleic acid according to [14], in which the 5' Cap is a Cap1 structure.
[16] The nucleic acid according to any one of [1] to [15], in which the protein-coding region encodes a hepatocyte growth factor.
[17] The nucleic acid according to [16], in which the hepatocyte growth factor is a human hepatocyte growth factor.
[18] The nucleic acid according to [17], in which the human hepatocyte growth factor includes an amino acid sequence selected from the group consisting of the following (i) to (iii):
   (i) an amino acid sequence set forth in SEQ ID NO: 2;
   (ii) an amino acid sequence in which one or a few amino acids in the amino acid sequence set forth in SEQ ID NO: 2 have been mutated; and
   (iii) an amino acid sequence having 80% or more sequence identity with the amino acid sequence set forth in SEQ ID NO: 2.
[19] The modified nucleic acid according to [17], in which a nucleotide sequence of the protein-coding region is a nucleotide sequence set forth in SEQ **ID** NO: 1.
[20] A drug composition containing the nucleic acid according to any one of [1] to [19].
[21] A drug composition for treating or preventing an ischemic disease, containing the nucleic acid according to any one of [16] to [19].
[22] The drug composition according to [21], in which the ischemic disease is chronic arterial occlusion, chronic obstructive pulmonary disease, interstitial pneumonia, acute lung injury, ophthalmic disease, optic nerve injury, or intractable skin ulcer.
[23] A method for producing a nucleic acid including a protein-coding region, the method including:
   (a) a step of determining a nucleotide sequence of the protein-coding region according to the following principles,
   (a1) codon-optimizing the protein-coding region for a cell into which the nucleic acid is to be introduced,
   (a2) selecting a codon to reduce a total proportion of uracil residues and adenine residues in the protein-coding region,
   (a3) in codons encoding the same amino acid, when alternatives for a first base of a codon are uracil and adenine, selecting a codon in which the first base is adenine, and
   (a4) in codons encoding the same amino acid, when alternatives for a third base of a codon include guanine and cytosine, selecting a codon in which the third base is guanine or cytosine and which is more frequently used in the cell; and
   (b) a step of producing a nucleic acid including the protein-coding region of the nucleotide sequence determined in the step (a).
[24] The method according to [23], in which, in the step (b), a modified uracil nucleotide is used as a uracil nucleotide.
[25] The method according to [24], in which the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.

### Examples

Hereinafter, the present invention will be described with reference to Examples, but the present invention is not limited to Examples.

### (Design of nucleotide sequence of human HGF coding region)

From a nucleotide sequence (SEQ **ID** NO: 3) of cDNA of human hepatocyte growth factor (HGF), the nucleotide sequence was modified based on the following principles.
(a) The amino acid sequence of human HGF (SEQ ID NO: 2) was not changed.
(b) The codons in the HGF coding region were changed to codons most frequently used in humans (codon optimization for humans).
(c) Codons including uracil and adenine were changed, as much as possible, to codons encoding the same amino acid and having a low proportion of uracil and adenine.
(d) In codons encoding the same amino acid, when there were a codon in which the first base was uracil and a codon in which the first base was adenine, the codon in which the first base was adenine was selected.
(e) In codons encoding the same amino acid, when there were a codon in which the third base was guanine and a codon in which the third base was cytosine, the codon more frequently used in humans was selected.

As the modified nucleotide sequence of human HGF, the nucleotide sequence set forth in SEQ ID NO: 1 was obtained. In SEQ ID NO: 1, "T" represents a uracil residue in RNA and a thymine residue in DNA.

### (Synthesis of mRNA)

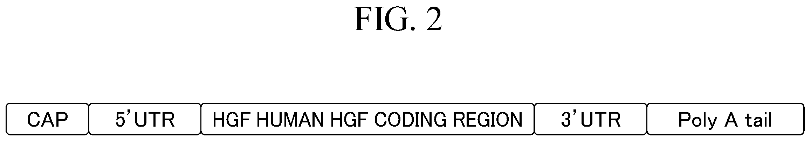

Using the HGF coding region whose nucleotide sequence was determined as described above as an ORF, mRNA synthesis was commissioned to TriLink BioTechnologies. In the synthesis of mRNA, 5-methoxyuridine-triphosphate was used as the uracil nucleotide. mRNA synthesis was performed by a short oligo priming method. For mRNA capping, CleanCap(R) AG (TriLink BioTechnologies) was used, and an mRNA (see FIG. 2) to which a Cap1 structure (see FIG. 1) had been added was synthesized.

Purification of the mRNA was performed by DNase treatment and oligo dT purification. The purified mRNA was dissolved in DNase-free and RNase-free water at a concentration of approximately 1 mg/mL, and dispensed in 500 µg portions. The mRNA was stored at -80°C and transported from the contractor on dry ice.

### (Evaluation of mRNA)

As controls for the synthesized mRNA (hereinafter, referred to as "AG-mRNA"), a comparative test was conducted using BioCap^{™} HGF mRNA (hereinafter, "BioCap"; phaRNA), Human HGF mRNA (hereinafter, "BioGene"; Creative BioGene), Collategene, which is plasmid DNA (hereinafter, "HGF Plasmid"; AnGes), and Naked HGF plasmid (hereinafter, "Naked HGF Plasmid").

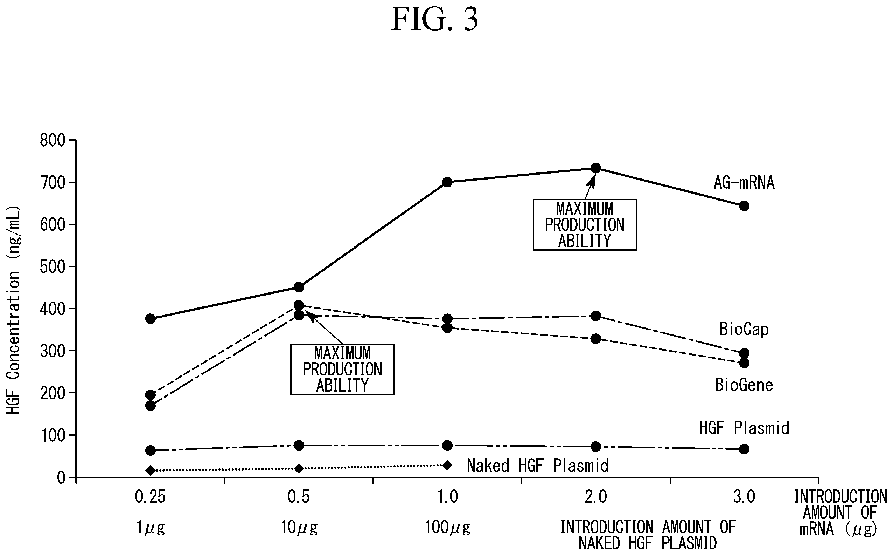

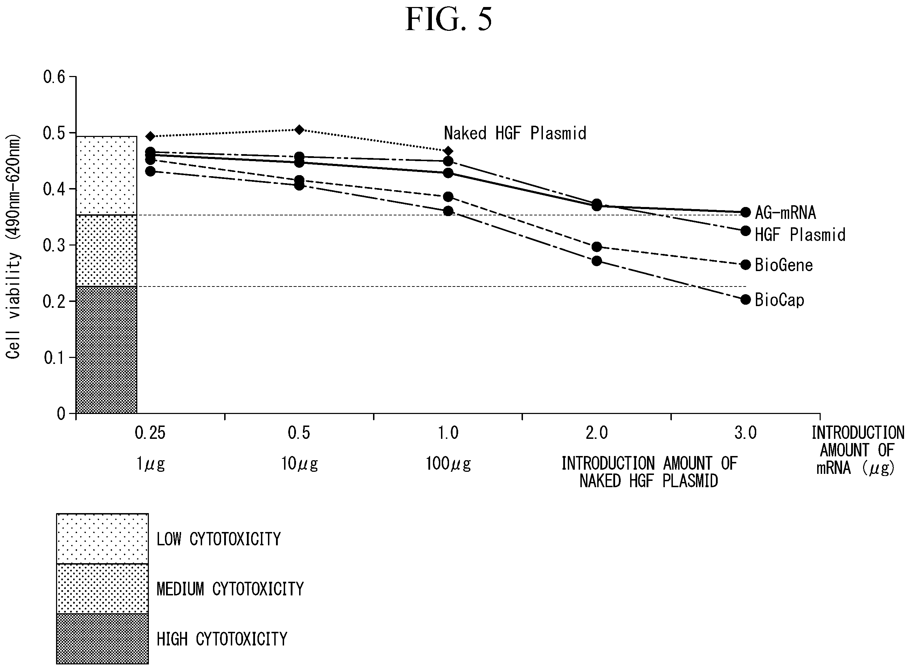

When the introduced nucleic acid was mRNA, introduction of the nucleic acid into cultured cells was performed using Lipofectamine Messenger MAX from Thermo Fisher Scientific according to the attached protocol. When the introduced nucleic acid was plasmid DNA, it was performed using Lipofectamine LTX from Thermo Fisher Scientific according to the attached protocol. Naked plasmid in FIGS. 3 and 5 indicates a case where the plasmid DNA was directly introduced without using an introduction reagent.

### HGF production amount:

IMR-90 (10⁵ cells/mL) after the AG-mRNA introduction was cultured at 37°C for 48 hours using Minimum Essential Media from Thermo Fisher Scientific. After the culturing, the culture solution was centrifuged, and the supernatant was collected. The amount of HGF in the supernatant was measured using HGF Human ELISA kit Quantikine from R&D Systems according to the attached protocol.

The results are shown in FIG. 3. It was confirmed that all mRNAs produced much higher HGF as compared with the HGF Plasmid and Naked HGF plasmid. Among the mRNAs, the highest HGF production was confirmed in AG-mRNA. The maximum HGF production amount (approximately 700 ng/mL) of AG-mRNA was confirmed at an introduction amount of 2 µg. In BioCap and BioGene, the maximum HGF production amount was confirmed at an introduction amount of 0.5 µg, and was approximately 400 ng/mL in both cases.

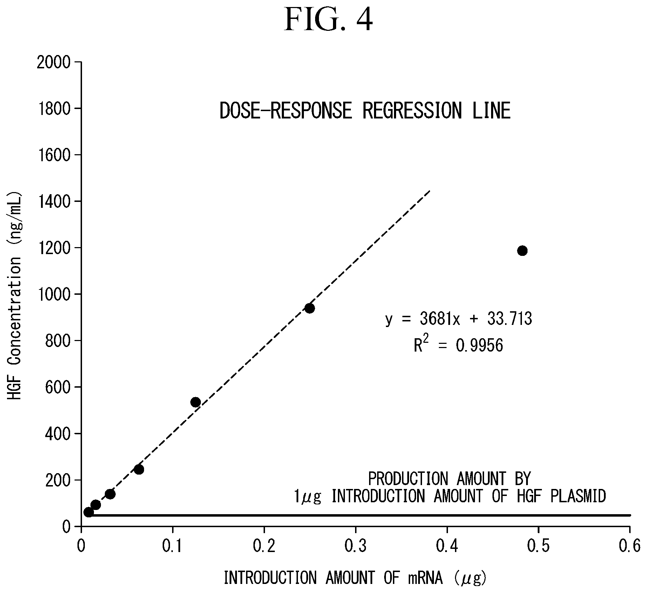

FIG. 4 shows a dose-response regression line prepared from the introduction amount of AG-mRNA and the HGF production amount. From the dose-response regression line, the introduction amount of AG-mRNA having an activity equivalent to 1 µg of HGF plasmid was 0.0130 µg. From the above, it was confirmed that AG-mRNA had an activity approximately 80 times that of HGF plasmid.

### Cell survival rate:

IMR-90 (10⁵ cells/mL) after each nucleic acid introduction was cultured at 37°C for 48 hours using Minimum Essential Media from Thermo Fisher Scientific. The cells after the culturing were collected, and a viable cell rate after each nucleic acid introduction was measured using Premix WST-1 Cell Proliferation Assay System from Takara Bio according to the attached protocol.

The results are shown in FIG. 5. In AG-mRNA, the cell survival rate was high and the cytotoxic activity was low as compared with BioGene and BioCap. It was considered that AG-mRNA and HGF Plasmid had a high cell survival rate and low cytotoxic activity.

### <Evaluation of duration of action>

### HGF production in continuous exposure test:

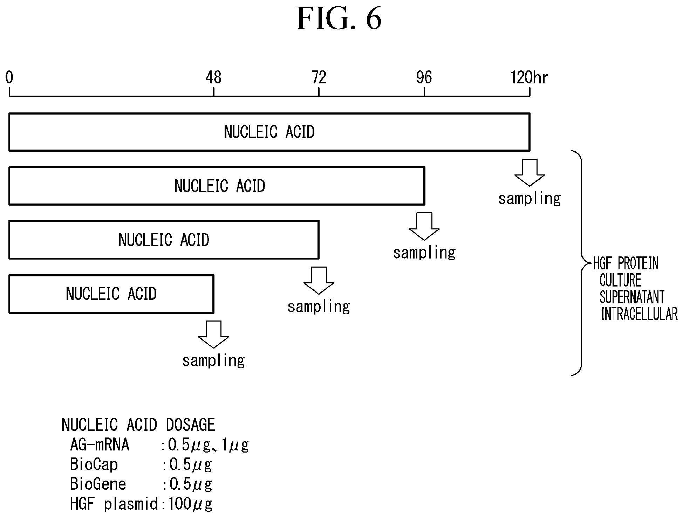

IMR-90 (10⁵ cells/mL) was cultured at 37°C using Minimum Essential Media from Thermo Fisher Scientific in the presence of 1 µg or 0.5 µg of AG-mRNA. The culture solution was sampled 48 hours, 72 hours, 96 hours, and 120 hours after the start of culture. The sampled culture solution was centrifuged to obtain a culture supernatant and cells. For each of the obtained culture supernatant and cells, the HGF concentration was measured using HGF Human ELISA kit Quantikine from R&D Systems according to the attached protocol (see FIG. 6).

As controls, the same test was performed using 0.5 µg each of BioCap and BioGene, and 100 µg of HGF Plasmid. As a negative control, IMR-90 was cultured without adding any mRNA or plasmid.

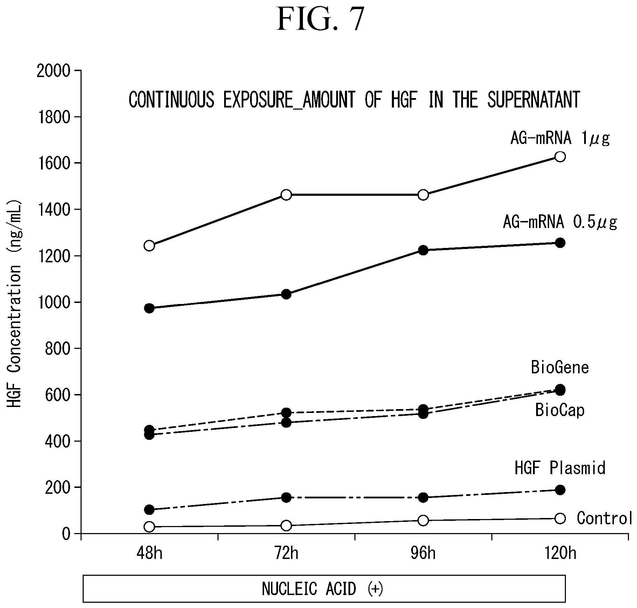

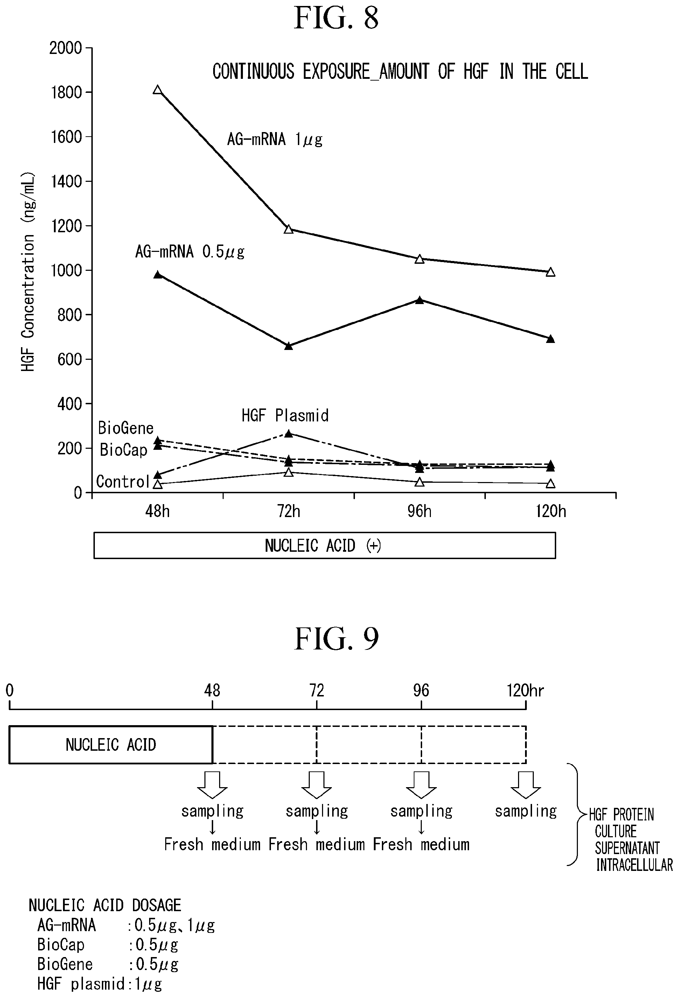

The results are shown in FIGS. 7 and 8. FIG. 7 shows the HGF concentration in the culture supernatant. FIG. 8 shows the HGF concentration in the cells.

When AG-mRNA was used, the HGF concentration in the culture supernatant was higher than when other mRNAs and HGF Plasmid were used. In the culture supernatant using AG-mRNA, the HGF concentration gradually increased up to 120 hours. In the culture supernatant of AG-mRNA, HGF was present at 48 hours at a concentration of 70% or more of the concentration at 120 hours (FIG. 7).

When AG-mRNA was used, the intracellular HGF concentration was higher than when other mRNAs and HGF Plasmid were used. In the cells using AG-mRNA, the HGF concentration tended to gradually decrease up to 120 hours. In the cells using AG-mRNA, a high HGF concentration was maintained even at 120 hours (FIG. 8).

### HGF production in 48-hour exposure test:

IMR-90 (10⁵ cells/mL) was cultured at 37°C for 48 hours using Minimum Essential Media from Thermo Fisher Scientific in the presence of 1 µg or 0.5 µg of AG-mRNA. Subsequently, the same medium not containing AG-mRNA was added in the same amount as the removed medium, and culturing was continued. The culture solution was sampled 48 hours, 72 hours, 96 hours, and 120 hours after the start of culture. For the sampled culture solution, the concentration of HGF was measured using HGF Human ELISA kit Quantikine from R&D Systems according to the attached protocol (see FIG. 9).

As controls, 0.5 µg each of BioCap and BioGene and 1 µg of HGF Plasmid were used, and the same test was conducted. As a negative control, IMR-90 was cultured without adding any mRNA or plasmid.

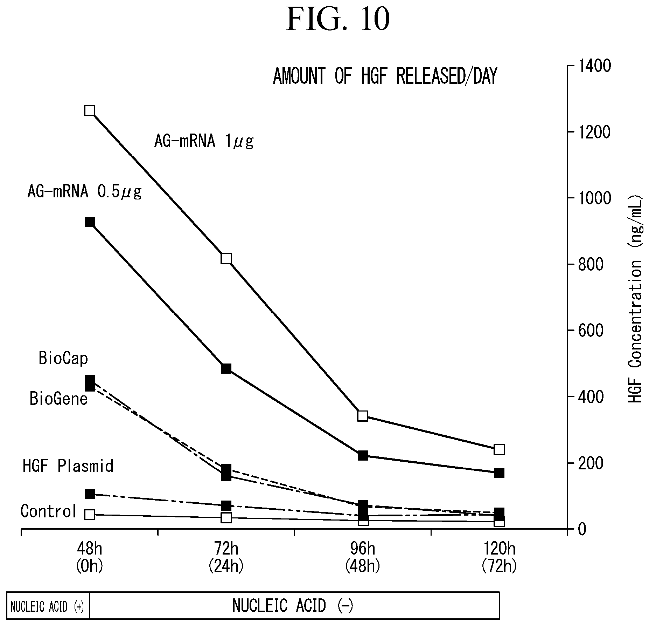

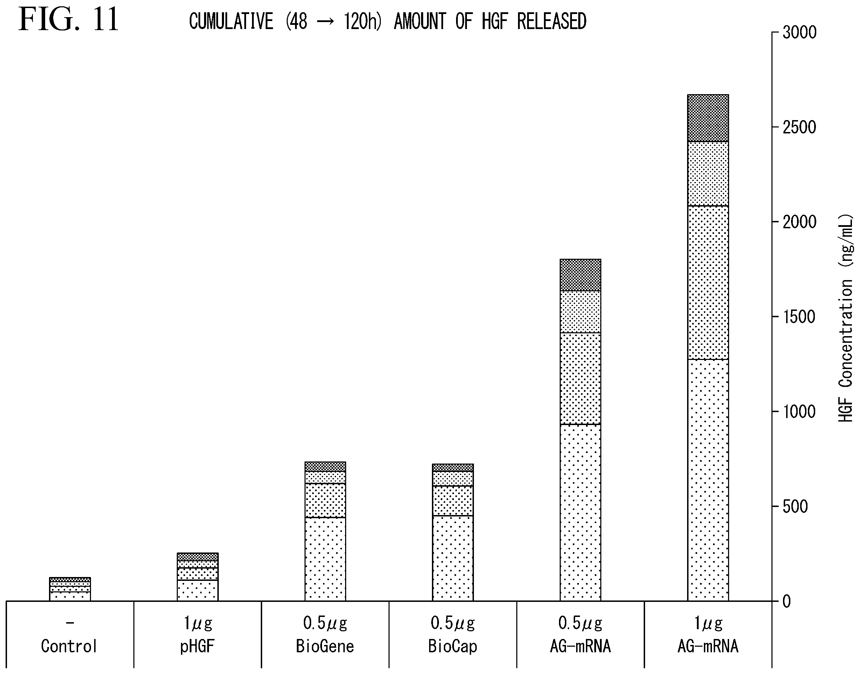

The results are shown in FIGS. 10 and 11. FIG. 10 shows the amount of HGF released per day from cells into the culture supernatant, calculated from the HGF concentration in the culture supernatant. FIG. 11 shows the cumulative amount of HGF released from cells into the culture supernatant from 48 hours to 120 hours of culture.

When AG-mRNA was used, the amount of HGF released from cells into the culture supernatant was greater than that when other mRNAs and HGF Plasmid were used (FIGS. 10 and 11). When AG-mRNA was used, although the amount of HGF released from cells into the culture supernatant decreased from 48 hours to 120 hours, HGF release continued even at 120 hours (FIGS. 10 and 11).

The cumulative amount of HGF released (48 → 120 hours) when AG-mRNA was used was approximately 10-fold at an introduction amount of 1 µg and approximately 7-fold at an introduction amount of 0.5 µg, as compared with HGF Plasmid.

After introduction of AG-mRNA, the maximum amount of HGF per day was released to the outside of the cells at 72 hours (3 days). The amount of HGF released gradually decreased after that, but HGF continued to be released to the outside of the cells even at 120 hours (5 days).

From the above-described results, it was found that AG-mRNA had lower cytotoxic activity and higher HGF production ability as compared with other HGF nucleic acids.

It was found that AG-mRNA exhibited a concentration-dependent increase in HGF production up to an introduction amount of 2 µg. At the tested introduction amounts, AG-mRNA showed the maximum HGF production ability (700 ng/mL) at an introduction amount of 2 µg. The minimum introduction amount at which HGF production was confirmed was 0.0130 µg (FIGS. 3 and 4).

For AG-mRNA, no significant decrease in cell viability was confirmed up to an introduction amount of 2 µg. On the other hand, for the control mRNAs of BioCap and BioGene, cell viability started to decrease from an introduction amount of 0.5 µg, and cell viability further decreased at an introduction amount of 1 µg (FIG. 5).

It was found that, by introduction of AG-mRNA, release of HGF produced in the cells to the outside of the cells continued for 120 hours or more. On the other hand, for HGF Plasmid, the duration was approximately 72 hours (FIGS. 10 and 11).

It was found that, after introduction of AG-mRNA, a certain amount of HGF was released from inside the cells to the outside of the cells up to 72 hours (FIGS. 10 and 11).

### INDUSTRIAL APPLICABILITY

According to the present invention, there are provided a nucleic acid having low cytotoxic activity and an improved production amount of a protein, a drug composition containing the nucleic acid, and a method for producing the nucleic acid.

Hereinabove, preferable examples of the present invention have been described above, but the present invention is not limited to these examples. Configurations can be added, omitted, and replaced, and other modifications can be made within a range not departing from the gist of the present invention. The present invention is not limited by the above description, but only by the scope of the appended claims.

## Claims

1. A nucleic acid comprising a modified protein-coding region,
wherein a total proportion of uracil residues and adenine residues in the modified protein-coding region is reduced relative to a corresponding protein-coding region before modification.

2. The nucleic acid according to Claim 1, wherein at least a portion of codons included in the protein-coding region before modification are replaced with codons encoding the same amino acid and having a lower proportion of uracil and adenine.

3. The nucleic acid according to Claim 1, wherein at least a portion of codons included in the protein-coding region before modification and having uracil as a first base are replaced with codons encoding the same amino acid and having adenine as a first base.

4. The nucleic acid according to Claim 1, wherein at least a portion of codons included in the protein-coding region before modification are replaced with codons encoding the same amino acid and more frequently used in a cell into which the nucleic acid is to be introduced.

5. The nucleic acid according to Claim 1, wherein at least a portion of uracil residues contained in the nucleic acid are residues derived from a modified uracil nucleotide.

6. The nucleic acid according to Claim 5, wherein the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.

7. The nucleic acid according to Claim 1, wherein the nucleic acid is an mRNA.

8. The nucleic acid according to Claim 1, wherein the protein-coding region encodes a hepatocyte growth factor.

9. The nucleic acid according to Claim 8, wherein a nucleotide sequence of the protein-coding region is a nucleotide sequence set forth in SEQ ID NO: 1.

10. A drug composition comprising the nucleic acid according to any one of Claims 1 to 9.

11. A drug composition for t^{r}eating or preventing an ischemic disease, comprising the nucleic acid according to Claim 8 or 9.

12. The drug composition according to Claim 11, wherein the ischemic disease is chronic arterial occlusion, chronic obstructive pulmonary disease, interstitial pneumonia, acute lung injury, ophthalmic disease, optic nerve injury, or intractable skin ulcer.

13. A method for producing a nucleic acid including a protein-coding region, the method comprising:
(a) a step of determining a nucleotide sequence of the protein-coding region according to the following principles (a1) to (a4),
(a1) codon-optimizing the protein-coding region for a cell into which the nucleic acid is to be introduced,
(a2) selecting a codon to reduce a total proportion of uracil residues and adenine residues in the protein-coding region,
(a3) in codons encoding the same amino acid, when alternatives for a first base of a codon are uracil and adenine, selecting a codon in which the first base is adenine, and
(a4) in codons encoding the same amino acid, when alternatives for a third base of a codon include guanine and cytosine, selecting a codon in which the third base is guanine or cytosine and which is more frequently used in the cell; and
(b) a step of producing a nucleic acid including the protein-coding region of the nucleotide sequence determined in the step (a).

14. The method according to Claim 13, wherein, in the step (b), a modified uracil nucleotide is used as a uracil nucleotide.

15. The method according to Claim 14, wherein the modified uracil nucleotide is at least one selected from the group consisting of 5-methoxyuridine-5'-triphosphate, 5-methyluridine-5'-triphosphate, pseudouridine-5'-triphosphate, N1-methylpseudouridine-5'-triphosphate, N1-methyl-2'-O-methylpseudouridine-5'-triphosphate, N1-methoxymethylpseudouridine-5'-triphosphate, N1-propylpseudouridine-5'-triphosphate, biotin-16-aminoallyluridine-5'-triphosphate, 2'-O-methyluridine-5'-triphosphate, 5-bromouridine-5'-triphosphate, 5-iodouridine-5'-triphosphate, 4-thiouridine-5'-triphosphate, and 1-thio-uridine-5'-triphosphate.
